# EUROPEAN PATENT APPLICATION

(11) **EP 4 365 594 A1**
(43) Date of publication of application: **08.05.2024**
(21) Application number: 22205157.5
(22) Date of filing: 02.11.2022
(51) Int. Cl.: G01N 33/68

(54) **COMBINATION OF BIOMARKERS OF UPCOMING DELIVERY**

(71) Applicant: Institut National de la Santé et de la Recherche Médicale, 75013 Paris (FR); Centre national de la recherche scientifique, 75016 Paris (FR); Université Paris Cité, 75006 Paris (FR); Assistance Publique Hôpitaux de Paris, 75004 Paris (FR)
(72) Inventor: MEHATS, Céline, 94250 GENTILLY (FR)
(74) Representative: Cabinet Beau de Loménie

(57) **Abstract**

The present invention relates to the prognosis and diagnosis of upcoming birth. More specifically, the invention relates to a new combination of biomarkers for upcoming birth that enables the accurate prognosis and diagnosis of upcoming delivery, and in particular enables the accurate prognosis and diagnosis of preterm delivery.

## Description

### FIELD OF THE INVENTION

The present invention relates to a combination of biomarkers, and particularly, although not exclusively, to a combination of biomarkers of upcoming delivery. The combination of biomarkers is useful for predicting upcoming delivery within N days, for example for distinguishing individuals at risk of experiencing delivery before 37 weeks of gestation at least 7 days before delivery or for predicting delivery within at least 7 days or for predicting the need of labor induction after 41⁺⁰ weeks of gestation because of prolonged gestation.

### BACKGROUND OF THE INVENTION

Preterm birth or preterm delivery is defined by birth that takes place before the completion of 37 weeks of gestation. It is estimated that over 15 million babies are born preterm annually worldwide. Globally, preterm delivery is one of the leading causes of death for children under the age of five with an estimated of one million preterm delivery-related deaths. Many of the survivors face a lifetime of challenging disabilities which include learning disabilities and visual and hearing problems. Although neonatology advances in the past decades have increased survival rates for preterm delivery, above 20% of preterm neonates will suffer at least one major disability including chronic lung disease, impaired mental development, cerebral palsy, deafness, or blindness.

There is a significant need to identify pregnant women who are at risk of preterm delivery. In the current paradigms, treatment for high-risk pregnancies involves prophylactic treatment, enhanced medical surveillance or close monitoring of the pregnancy, which reduces preterm delivery rates. However, in most cases, classification of high-risk pregnancies is based on prior medical history or clinical examinations, identifying only a small subsection of the true high-risk pregnancies prone to preterm delivery. Still, most pregnancies that are prone to preterm deliveries are not identified at early stages and hence early medical intervention for such cases is not possible.

There are several tests in the market for risk assessment of preterm delivery for women with risk symptoms.

One such example is the Fetal Fibronectin (fFN) test which provides a risk assessment for symptomatic women. Fetal fibronectin is present in vaginal fluid if a preterm delivery is likely to occur. Hence, the fFN test is commonly used in pregnant women with symptoms indicating a possibility for preterm delivery, such as contractions, vaginal bleeding, vaginal fluid losses, increased vaginal discharge, backache and cramp in lower abdomen. The strength of the fFN test lies in its high specificity, that is to say its negative predictive value for up to 10 days following the test (i.e., a negative result means that there is a low possibility of preterm labor within the next 7 to 10 days following the test). However, when the fFN test is positive, the results are less conclusive since the sensitivity of the fFN test is not sufficient.

As a consequence, practitioners making use of this test cannot rely on a negative test result and are prone to correct negative test results by taking therapeutic and/or prophylactic for women having negative test results.

Another immunoassay test detecting Insulin-like Growth Factor-Binding Protein-1 (IGFBP-1) or Placental Alpha 1-Microglobulin (PAMG-1) is available on the US market. This immunoassay test is particularly useful for rupture of membranes diagnosis. Indeed, PAMG-1 and IGFBP1 are naturally present in high concentrations in amniotic fluid. The presence of high concentrations of PAMG-1 and IGFBP1 in vaginal fluid will result of a rupture of membranes, and thus of an impending preterm delivery.

Due to the risk of false negative results with the fFN test and to the only short anticipation allowed by the PAMG-1 and IGFBP1 tests, there is still a need of a preterm delivery test having enhanced prediction capabilities.

Labor, whether preterm or at term or post term, is now recognized as local inflammation in the uterus and cervix, placenta and chorioamniotic membranes *(*R. Romero, S. K. Dey, S. J. Fisher, Science. 345, 760-5 (2014*)).* The most commonly accepted hypothesis about inflammation in parturition is that of uterine activation in response to hormonal or paracrine mediators (cytokines, growth factors, etc.) or extracellular vesicles of fetal and/or maternal origin that spread to immune cells (R. Menon, E. A. Bonney, J. Condon, S. Mesiano, R. N. Taylor, Hum. Reprod. Update. 22, 535-560 (2016*)).* Besides, gene expression varies mostly in the maternal decidua (uterine mucosa) and fetal chorion (outermost layer of the membranes) (R. Bukowski et al., PeerJ. 5, e3685 (2017*)*)*,* a major maternal-fetal interface where maternal stromal and immune cells and fetal cells (primarily trophoblasts) intermingle.

In consequence, researchers have sought to explore the field of biomarkers of such local inflammation. However, a very high number of inflammation biomarkers and an even higher number of combination of biomarkers can be associated with preterm delivery, rendering the systematic evaluation of all these combinations impossible without human preselection.

To date, several combinations of biomarkers for preterm delivery are known.

For example, the IGFBP1-IL6 combination of biomarkers (on which the Premaquick^{®} test is based) is known of the person skilled in the art and one can show on Table 1 that even if the test has a satisfactory specificity (permitting high confidence in a negative result), the sensibility of this test is not sufficient, with 20% of false positive results.

WO2022/090050 also discloses a combination of biomarkers, in particular of up to ten biomarkers, for early prognosing and/or diagnosing a risk of preterm delivery. This combination comprises at least COL1A1 and CCN5, which are associated with extracellular matrix remodeling, and makes it possible to predict preterm delivery within 7 days after test even before 32 weeks of gestation.

However, the clinical evaluation of such tests for preterm delivery is a major challenge, since forming of a statistically significant cohort requires to recruit enough women who were hospitalized for preterm labor, practicing the test on these women and obtaining - a posteriori - among these women enough women who will effectively deliver within N days after test.

Consequently, the combinations of WO2022/090050 were evaluated on a cohort available before the filing date, which is to say a so-called first cohort of 105 women comprising only 5 women who effectively delivered before term and within 7 days after test. This cohort is the first cohort of example 1 of the present application.

Even if this first cohort was a first major step for evaluating the combination of biomarkers of WO2022/090050 and showed that the COL1A1-CCN5 based combinations were an improvement compared to prior art, there was a need of further clinical studies to investigate the field of delivery biomarkers.

Thanks to numerous recent clinical analyses, the inventors of the present patent application had the opportunity to evaluate the COL1A1-CCN5 combination and other prior art biomarkers on a so-called second cohort of 125 women comprising 30 women who effectively delivered before term and within 7 days after test (second cohort of example 1 of the present application), rendering further and more accurate statistical analysis possible.

This second evaluation, the results of which are presented on Table 1, showed that:
- the COL1A1-CCN5 combination predicted spontaneous delivery within 7 days after test day with a sensibility of 73.3% and a specificity of 67.8%, the delivery occurring despite usual treatments to prevent preterm birth;
- the IGFBP 1-IL6 combination predicted spontaneous delivery within 7 days with a sensitivity of 80,0% and a specificity of 90,0%.

In this case, the sensitivity is too low (first case) or still improvable (second case) such that even if the test result is negative, practitioners won't be confident in this result and will be prone to overreact. Thus, this second evaluation showed that there were still opportunities for identification of pregnancies with high-risk of preterm delivery with both sufficient sensibility and sufficient specificity and with a limited number of biomarkers.

The invention aims at proposing such a combination of biomarkers for predicting incoming delivery with both high sensitivity and specificity. In particular, the present invention aims at proposing a risk assessment for classification of women with high-risk of preterm delivery several days before symptoms of preterm delivery appear with a maintained or increased sensibility and a higher specificity compared to prior art.

### SUMMARY OF THE INVENTION

Thus, the invention relates to a combination of biomarkers comprising CCL2 and CEACAM1 for use as biomarkers of upcoming delivery of a pregnant individual within N days of a test day (TD), N being a strictly positive integer, optionally ranging from 7 to 14.

Such a combination of biomarkers allows to predict upcoming delivery within N days of a test day, N being possibly comprised between 7 and 14. The present invention allows for accurate, rapid and sensitive prognosis and diagnosis of upcoming delivery within N days through a measurement of a combination of specific biomarkers taken from sample of an individual at a single point in time, which helps anticipate decision making for pregnant women having a risk of preterm delivery and/or for pregnant women having not delivered at term and/or for pregnant women at term. The pair of indicators (sensitivity, specificity) characterizing this combination is notably enhanced compared to prior art. Therefore, the prediction is reliable and can be made used of:
- by practitioners to make a decision on a therapy against preterm delivery and/or on a therapy for the foetus in case the preterm delivery finally occurs,
- by practitioners to make a decision on labour induction, in case of possible post term delivery
- by practitioners to organise a maternity staff and/or by women to organize their way of life shortly before delivery.

In a particular embodiment, the invention relates to a combination of biomarkers comprising CCL2 and CEACAM1 for use as biomarkers of upcoming delivery of a pregnant individual within N days of a test day (TD), N being a strictly positive integer, optionally ranging from 7 to 14 for its use for an in vivo diagnostic/prognostic method.

In a particular embodiment, the invention relates to a combination of biomarkers comprising CCL2 and CEACAM1 for use as biomarkers of upcoming delivery of a pregnant individual within N days of a test day (TD), N being a strictly positive integer, optionally ranging from 7 to 14 for its use for an in vitro diagnostic/prognostic method.

In a particular embodiment, the combination of biomarkers further comprises one or more additional biomarkers of upcoming delivery selected from the group comprising IL1RL1, CD14, TNFRSF8, CCL18, CD163 and TGFα.

This renders the test more difficult to implement, since it requires strictly more than two biomarkers but one or more of these additional biomarkers help improve the pair (sensitivity, specificity) characterizing the predicting ability of the test.

In a particular embodiment, the one or more additional biomarkers of the combination of biomarkers comprise IL1RL1, CD14, TNFRSF8, CCL18, CD163 and TGFα. This combination of eight biomarkers is the one that has the best combination of sensitivity and specificity and can be interesting when a test that is more time and/or money consuming can be acceptable with respect to the test accuracy required.

In a particular embodiment, the combination comprises neither COL1A1 nor CCN5.

Indeed, the inventors have shown that adding the biomarkers COL1A1 and CCN5 to the combination of biomarkers according to the invention results in an increase of the confusion, or in other words in a decrease of the specificity and possibly of the specificity of the prediction ability of the combination of biomarkers.

The invention also relates to the use of a combination of biomarkers for prognosing and/or for diagnosing upcoming delivery of a pregnant individual within N days of a test day (TD), N being a strictly positive integer, optionally ranging from 7 to 14, wherein the combination of biomarkers comprises CCL2 and CEACAM1.

In a particular embodiment, the invention relates to the use in vitro of a combination of biomarkers for prognosing and/or for diagnosing upcoming delivery of a pregnant individual within N days of a test day (TD), N being a strictly positive integer, optionally ranging from 7 to 14, wherein the combination of biomarkers comprises CCL2 and CEACAM1.

In a particular embodiment, the invention relates to the use in vivo of a combination of biomarkers for prognosing and/or for diagnosing upcoming delivery of a pregnant individual within N days of a test day (TD), N being a strictly positive integer, optionally ranging from 7 to 14, wherein the combination of biomarkers comprises CCL2 and CEACAM1

In a particular embodiment, the invention relates to the use of a combination of biomarkers for prognosing and/or for diagnosing upcoming delivery of a pregnant individual within N days of a test day (TD), N being a strictly positive integer, optionally ranging from 7 to 14, wherein the combination of biomarkers comprises CCL2 and CEACAM1 and further comprises one or more additional biomarkers of upcoming delivery selected from the group comprising IL1RL1, CD14, TNFRSF8, CCL18, CD163 and TGFα.

In a particular embodiment, the invention relates to the use of a combination of biomarkers for prognosing and/or for diagnosing upcoming delivery of a pregnant individual within N days of a test day (TD), N being a strictly positive integer, optionally ranging from 7 to 14, wherein the combination of biomarkers comprises CCL2 and CEACAM1, and also IL1RL1, CD14, TNFRSF8, CCL18, CD163 and TGFα.

The invention also concerns a method for prognosing and/or for diagnosing upcoming delivery of a pregnant individual within N days of a test day (TD), N being a strictly positive integer, optionally ranging from 7 to 14, the method comprising:
a) determining at test day the presence and/or amount of a combination of biomarkers of upcoming delivery in a sample obtained from the individual, and
b) comparing the presence and/or amount of each biomarker of said combination of biomarkers obtained from step a) to the presence and/or amount of each biomarker of said combination of biomarkers in a control sample, to identify an increased risk for preterm delivery,
wherein said combination of biomarkers of upcoming delivery comprises CCL2 and CEACAM1.

In a particular embodiment of the method for prognosing and/or for diagnosing upcoming delivery of a pregnant individual within N days of a test day (TD), the sample is a sample of vaginal fluid.

In a particular embodiment of the method for prognosing and/or for diagnosing upcoming delivery of a pregnant individual within N days of a test day (TD), the combination of biomarkers of upcoming delivery of step a) further comprises one or more additional biomarkers for upcoming delivery selected from the group comprising IL1RL1, CD14, TNFRSF8, CCL18, CD163 and TGFα.

In a particular embodiment of the combination of biomarkers according to the preceding embodiments, or of the use of a combination of biomarkers according to the preceding embodiments or of the method for prognosing and/or for diagnosing upcoming delivery of a pregnant individual within N days of a test day (TD) according to the preceding embodiments, the biomarkers are protein or nucleic acid encoding for said protein.

In a particular embodiment of the method for prognosing and/or for diagnosing upcoming delivery of a pregnant individual within N days of a test day (TD), the presence and/or amount of the CCL2, respectively CEACAM1, biomarker is determined using an antibody and/or an oligonucleotide specific for said CCL2, respectively CEACAM1, biomarker, and the presence and/or amount of the one or more additional biomarkers for upcoming delivery is determined using an antibody and/or an oligonucleotide specific for said one or more additional biomarkers for upcoming delivery.

In a particular embodiment of the method for prognosing and/or for diagnosing upcoming delivery of a pregnant individual within N days of a test day (TD), one more a) and one additional b) are performed at least at one time point (PTD) before test day (TD).

Performing at least two sample analysis allows to measure the evolution of the amount of the biomarkers of the combination over time, which provides additional information to a unique sample and can help make a better decision regarding the pregnant individual than with a single test.

In a particular embodiment of the use of a combination of biomarkers according to the preceding embodiments or of the method for prognosing and/or for diagnosing upcoming delivery of a pregnant individual within N days of a test day (TD) according to the preceding embodiments, test day (TD) is comprised either at least N days before 37 weeks minus 1 day and upcoming birth is preterm birth ; test day (TD) is comprised between N days before 37 weeks and N days before 41 weeks and 6 days and upcoming birth is term birth from 7 to 14 ; or test day (TD) is strictly after N days before 41 weeks and 6 days and upcoming birth is post term birth.

A test day (TD) at least N days before 37 weeks minus 1 day is adapted to diagnose/prognose preterm delivery. A test day (TD) comprised between N days before 37 weeks and N days before 41 weeks and 6 days is adapted for making decisions relative to term birth. A test day (TD) strictly after N days before 41 weeks and 6 days and upcoming birth is adapted for making decisions relative to post term birth.

Last, the invention deals with the use of a device for carrying out the use according to any of the preceding embodiments or for carrying out the method according to any one of the preceding embodiments, which comprises:
a) one or more antibody and/or oligonucleotide specific for the combination of CCL2 and CEACAM1 biomarkers of upcoming delivery, and optionally
b) one or more antibody and/or oligonucleotide specific for any one combination of any of the one or more additional biomarkers of upcoming delivery selected from the group comprising IL1RL1, CD14, TNFRSF8, CCL18, CD163 and TGFα, and optionally
c) at least one internal standard.

In a particular embodiment the use of the preceding device is performed in vitro.

### DESCRIPTION OF TABLES

Table 1 shows the sensitivity (true positive rate) and the specificity (true negative rate) of prior art biomarkers combinations on cohort 1 and/or cohort 2 of example 1 for N = 7 days.
Table 2a shows the sensitivity and the specificity of biomarkers combinations according to the invention and comparative biomarkers combination on cohort 2 of example 1 for N = 7 days.
Table 2b shows the sensitivity and the specificity of biomarkers combinations according to the invention and comparative biomarkers combination on cohort 1 of example 1 for N = 7 days.
Table 3 shows the sensitivity and the specificity of biomarkers combinations according to the invention on cohort 2 of example 1 for N = 7 days to N = 14 days.

### DESCRIPTION OF FIGURES

Figure 1 shows the results of differentially expressed gene analysis for six maternal and fetal cells subpopulation in terms of False Discovery Rate (FDR).
Figure 2 is a diagram illustrating the values of the internal standard at the time of sampling in women with symptomatic preterm labor as a function of the term of spontaneous delivery.
Figure 3 is a graph showing the number of biomarkers having a Z-score higher than 1 in subjects according to gestational age at admission and delay until delivery, in the case of cohort 1 of example 1.

### DETAILED DESCRIPTION

The present invention allows for the rapid, sensitive, and accurate diagnosis or prognosis of an upcoming delivery using one or more samples obtained from an individual at a single time point ("snapshot") or repeatedly.

As used herein, the expression "upcoming childbirth" or "upcoming birth" or "upcoming delivery" may be used to refer to a delivery which will occur within less than or equal to N days after test day TD, N being a strictly positive integer.

In general, a test according to the invention is performed at test day TD and allows to predict delivery within N days after test day TD, N ranging in a particular embodiment from 7 days to 14 days, and being in particular embodiment equal to 7, 8, 9, 10, 11, 12, 13 or 14.

Thanks to the invention, in a first use case, preterm birth or preterm delivery may be diagnosed or prognosed prior to the onset of clinical symptoms, and/or as subsequent confirmation after the onset of clinical symptoms.

As used herein "preterm birth" or "preterm delivery" includes the delivery of a baby prior to full gestation. For example, delivery of the baby less than 37 weeks of gestation is considered a preterm delivery. The term preterm delivery is synonymous with preterm delivery and premature delivery. "Preterm birth" or "preterm delivery" can be used interchangeably.

In this first case, the test is performed at a test day TD at least N days up to 37 weeks minus 1 day.

In this first case, the present invention allows for more effective therapeutic or prophylactic intervention and/or diagnosis in the pre-symptomatic stage.

In case of a preterm delivery prognosis or diagnostic, practitioners can take therapeutic and/or prophylactic actions for the mother to be (drug therapy, transfer to a specialized maternity hospital/unit,...) and/or prophylactic actions for the foetus (corticoid therapy, magnesium sulfate therapy,...).

The invention also allows to prognose upcoming delivery in the case of a term labor. In this second case, the test is performed at a test day TD between N days before 37 weeks and N days before 41 weeks and 6 days.

In this second case, by way of example, the invention can be made used of for organizing the staff at the maternity staffs and/or for pregnant women to adapt their way of life in the last days before delivery, in particular the last N days before delivery.

Post term delivery requiring induction of labor may also be prognosed/ diagnosed thanks to the invention.

In this third case, the test is performed at a test day TD strictly after N days before 41 weeks and 6 days. Such a test can be implemented in order to decide if there is a hope of spontaneous labor even after 41 weeks and 6 days, in particular in one, two, three, four, five or six days after 41 weeks and 6 days. In case of a positive result, some labor induction and/or caesarean section could be prevented or on the contrary, some labor induction and/or caesarean section could be decided earlier than without a test according to the invention.

More precisely, the present invention relates to a combination of biomarkers comprising CCL2 and CEACAM1 for use as biomarkers for an upcoming delivery within N days, N being a strictly positive integer, and N ranging in a particular embodiment from 7 to 14 days.

As used herein, the term "combination" may be used to refer to a mixture of at least two different biomarkers. The combination may comprise at least three different biomarkers, at least four different biomarkers, at least five different biomarkers, at least six different biomarkers, at least seven different biomarkers, at least eight different biomarkers.

As used herein, the term "biomarker" or "biomarkers" may be used to refer to a naturally occurring biological molecule present in a sample obtained from pregnant individual at varying concentrations and that may be isolated from, or measured in the sample, and which is particularly useful in prognosis and/or diagnosis upcoming delivery, advantageously useful in prognosis and/or diagnosis the risk of preterm delivery.

As used herein, an "individual" is an animal, advantageously a mammal, more advantageously a human. The terms "individual", "subject" and "patient" are used interchangeably herein.

For example, the biomarker can be a protein and a fragment thereof, a peptide, a polypeptide, a proteoglycan, a glycoprotein, a lipoprotein, a carbohydrate, a lipid, a nucleic acid, an organic on inorganic chemical, a natural polymer, and a small molecule. Furthermore, a biomarker can be the entire intact molecule, or it can be a portion thereof that may be partially functional or recognized, for example, by an antibody or other specific binding protein.

A biomarker is considered to be informative if a measurable aspect or characteristic of the biomarker is associated with a given state of an individual, such as in this case preterm delivery or imminent birth. Such a measurable aspect or characteristic may include, for example, the presence, absence, amount or concentration of the biomarker in the biological sample from the individual and/or its presence as part of a profile of biomarkers. Such a measurable aspect of a biomarker is defined herein as a "feature." For example, the presence of a biomarker may be a feature. As another example, the amount of a biomarker in a sample, or the amount of a biomarker in a sample compared with a control or reference sample may be a feature. A feature may also be a ratio of two or more measurable aspects of biomarkers, which biomarkers may or may not be of known identity, for example.

The biomarkers disclosed herein were first identified in a meta-data analysis, and subsequently demonstrated in patient-derived samples.

The inventors have performed extensive clinical experiments that collectively suggest that the fetal stromal microenvironment surrounding trophoblasts promotes immune suppression at the choriodecidual interface in late pregnancy, but switches toward an inflammatory state with labor onset, expressing potent immuno-regulator signals.

In particular, they used a single-nucleus RNA-seq (snRNA-seq) approach to effectively interrogate the molecular changes at the maternal-fetal interfaces accompanying labor. Nuclei from 14 choriodeciduas and 6 placentas collected between 37-42 gestational weeks in two groups were isolated: one group with spontaneous labor and vaginal deliveries (TIL, term in labor) and the other one with planned prelabour caesarean deliveries, which are deliveries without labor (TNL, term not in labor). Using a droplet-based encapsulation technology, snRNA-seq libraries were generated.

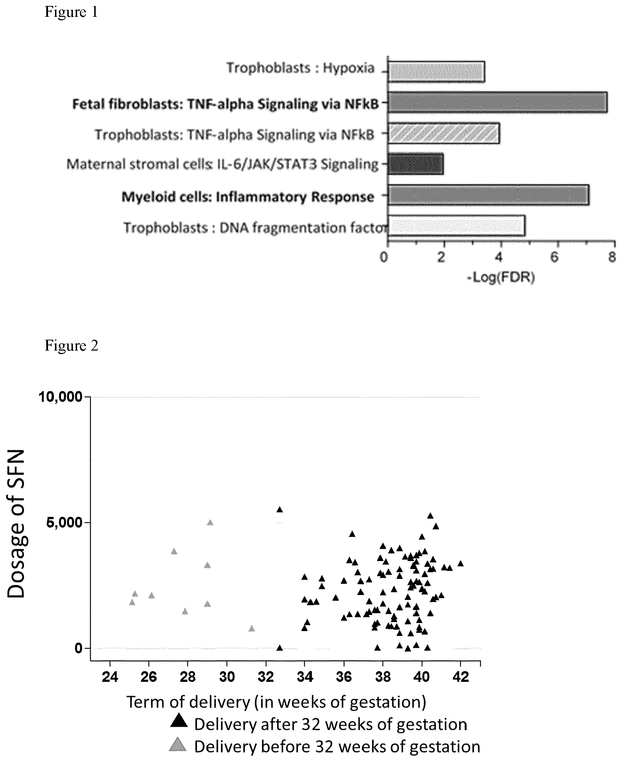

The choriodecidual and placental final datasets comprised around 60 000 nuclei and around 32 000 nuclei, respectively. The following cell types were identified: trophoblasts, stromal cells, structural cells (lymphatic and vascular endothelial cells and epithelial cells) and immune cells - mostly of myeloid lineage - in choriodecidua and placental villi. Differentially Expressed Gene (DEG) analyses between TIL and TNL samples were performed as shown on Figure 1.

This study allowed to conclude that:
- foetal fibroblasts and trophoblasts in choriodecidua are the most transcriptionally modified subpopulations by labour and foetal fibroblasts (- log(False Discovery Rate) = 8),
- and that myeloid cells showed the highest proinflammatory phenotype (- log(False Discovery Rate) = 7).

Further, adherent cells isolated from choriodecidua with or without labor were cultured by regularly renewing the culture medium and a bulk RNA-seq analysis at confluence was then performed. Term enrichment analysis showed that genes associated with inflammation and allograft rejection were overrepresented in labor-derived cells. A metacluster of activated myeloid cells present only in labor-derived cultures was identified.

The persistence of differences in myeloid phenotypes observed in the fibroblast microenvironment implies profound intrinsic changes after the onset of labor.

In view of these results, 30 Differentially Expressed Genes (DEGs) coding for secreted proteins were filtered, based on a combination of *P*-values, fold changes, and functional annotation related to immunity, inflammation, for downstream screening in cervicovaginal fluids (CVFs).

Based on these 30 DEG's, biomarkers that are related to inflammation were identified as additional biomarkers to be potentially combined with the two biomarkers of extracellular matrix modifications COL1A1 and CCN5, as disclosed in WO/2022/0900508: CCL2, IL1RL1, CD14, CD30, CCL18, CD163, TGFα and CEACAM1.

However, recent studies emphasised that changes in immunity are more preeminent than extracellular matrix modifications during labor.

In particular, crosstalk between stromal, trophoblast, and immune cells was studied, leading to the inference of 139 genes associated with ligand activity on myeloid cells. The top Gene Ontology terms characterizing these genes are pathways in cancer and inflammation. Notably, 7 of the 10 top-ranked ligands originated from foetal fibroblasts and included CSF1, a cytokine that controls macrophage differentiation and is highly expressed postlabor. The publicly available repository of ligands, receptors, and their interactions CellPhoneDB was also used allowing to find novel putative ligand-receptor interactions between fetal fibroblasts and myeloid cells in women who laboured, related to inflammation.

These analyses emphasised that, unexpectedly, changes in inflammation are more preeminent that extracellular matrix modifications during labor, hinting that combinations of biomarkers which are not based on extracellular matrix biomarkers such as the COL1A1-CCN5 combination but rather on inflammation biomarkers could be considered and promising.

As a consequence, the inventors re-explored the set of 30 Differentially Expressed Genes previously filtered with a completely new mindset and with a statistical evaluation based on cohort 2 of example 1. These extensive analyses allowed to conclude that the combination of the two biomarkers CCL2 and CEACAM1 was the one allowing, either alone or in combination, both the highest specificity and the highest sensitivity as shown on Table 2a.

Biomarkers disclosed herein differentiate samples from individuals delivering within N days after test day TD from samples from individuals not delivering within N days after test day TD, even without clinical symptoms or signs.

Such biomarkers may in particular be useful for identifying an individual at risk of preterm delivery, and thus may be useful for guiding clinical decisions such as the initiation of treatment to prolong gestation and/or prevent or reduce the risk of preterm delivery. Advantageously, the biomarkers are proteins or nucleic acids issued from motherly tissues and not from the foetal tissue.

In one embodiment, the biomarker can be a protein or a nucleic acid encoding for a protein present in higher or lower amounts in an individual at risk of preterm delivery relative to the amount of the same biomarker in an individual who did not experience preterm delivery. In advantageously embodiment, the biomarker is a protein or a nucleic acid encoding for a protein.

As disclosed herein, CCL2 and CEACAM1 are biomarkers of upcoming delivery.

As used herein "CCL2" may refer to the C-C motif chemokine ligand 2 gene or to the C-C motif chemokine ligand 2 protein. According to the present invention, "CCL2" or "MCP1" or "chemokine (C-C motif) ligand 2" or "monocyte chemoattractant protein-1" or "monocyte chemotactic and activating factor" or "monocyte chemotactic protein 1" or "monocyte secretory protein JE" or "small inducible cytokine A2 (monocyte chemotactic protein 1, homologous to mouse Sig-je)" or "small inducible cytokine subfamily A (Cys-Cys) member 2" or "small-inducible cytokine A2" can be used interchangeably. GDCF-2, HC11, HSMCR30, MCAF, MCP-1, MCP1, SCYA2, SMC-CF and C-C motif chemokine ligand 2 can be used interchangeably.

As used herein CEACAM1 (CarcinoEmbryonic Antigen-related Cell Adhesion Molecule 1) is the gene name that refers to the biliary glycoprotein ("BGP", sometimes also called Biliary glycoprotein D or MHVR1 or Murine hepatitis virus receptor (MHV-R)), and may also be called by the antigen name "CD66a" (Cluster of Differentiation 66a).

Surprisingly, the inventors have shown that the increased amount of the specific combination of biomarkers, and in particular the increased amount of the combination of CCL2 and CEACAM1 biomarkers is the most promising for indicating with higher confidence level than prior art combinations that delivery is upcoming within N days after test day.

In particular, as can be observed on Table 2a, the CCL2-CEACAM1 combination allows when evaluated on cohort 2:
a) much higher specificity than CEACAM1 alone and higher specificity than CCL2 alone, with the same sensitivity.
   This proves that the combination of CCL2 and CD66 a is an improvement compared to each biomarker on its own.
b) much higher sensitivity and higher specificity than the combination COL1A1-CCN5 alone and much higher specificity than when combined with COL1A1-CCN5, showing that the predictive abilities of a combination based on COL1A1-CCN5 are poorer than the ones of a combination based on CCL2-CEACAM1. These results are confirmed by the comparison of the combination of the eight biomarkers: CCL2 - CEACAM1 and IL1RL1, CD14, TNFRSF8, CCL18, CD163 and TGFα without or with COL1A1-CCN5 (two last lines of Table 2a).

We can thus assert that the CCL2-CEACAM1 combination as higher predictive capabilities than the COL1A1-CCN5 combination and that the COL1A1-CCN5 combination increases confusion when combined with a biomarker combination based on the CCL2-CEACAM1 combination.

As used herein "COL1A1" may refer to the collagen type I alpha 1 chain gene or to the collagen alpha-1(1) chain protein. According to the present invention, "COL1A1" or "collagen type I alpha 1 chain" or "alpha-1 type I collagen" or "alpha1(I) procollagen" or "collagen alpha 1 chain type I" or "collagen alpha-1(I) chain preproprotein" or "collagen of skin, tendon and bone" or "pro-alpha-1 collagen type 1" or "type I procollagen alpha 1 chain" can be used interchangeably.

As used herein "CCN5" may refer to the cellular communication network factor 5 gene or to the CCN family member 5 protein. According to the present invention, "CCN5" or "WISP2" or "WNT1 inducible signaling pathway protein 2", or "connective tissue growth factor-like protein" or "connective tissue growth factor-related protein 58" can be used interchangeably.
c) much higher sensitivity, even if lower specificity than when combined with any of the prior art biomarkers IGFBP1 and IGFBP1-IL6 or the same sensitivity but much higher specificity than when combined with prior art biomarker fFN.

This further shows that not any combination based on the CCL2-CEACAM1 combination is an improvement compared to prior art or to the CCL2-CEACAM1 combination alone.

These results can be compared to the ones for the combination of the eight following biomarkers: CCL2 - CEACAM1 and IL1RL1, CD14, TNFRSF8, CCL18, CD163 and TGFα. As a conclusion, the choice of additional biomarkers is a tricky one, all the more that multiplying the number of biomarkers renders the implementation of the test difficult and that to date, only tests based on less than ten biomarkers simultaneously can be implemented.

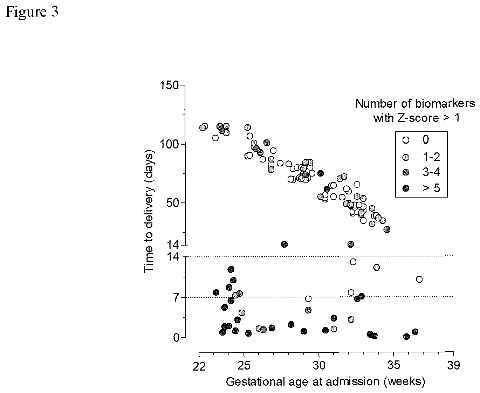

Figure 3 helps understand the challenge associated with finding a combination comprising a limited number of biomarkers. Figure 3 shows that in cervico-vaginal fluids of subjects who delivered within 7 days, regardless of gestational age at admission, at least five biomarkers had a Z-score more than 1 while most of the individuals who didn't deliver within 7 days had less than 2 biomarkers having a Z-score more than 1.

The predictive abilities of the CCL2-CEACAM1 combination and of combination of the eight following biomarkers: CCL2 - CEACAM1 and IL1RL1, CD14, TNFRSF8, CCL18, CD163 and TGFα, when evaluated on cohort 1 of example 1 are provided on Table 2b only for informational purposes. Even if cohort 1 example 1 is statistically less significant than cohort 2 of example 1, one can observe that there is no contradiction between the results of the two cohorts concerning these combinations.

As a conclusion, the invention relates to:
- the CCL2-CEACAM1 combination,
- and optionally a combination of one or more of the six additional biomarkers IL1RL1, CD14, TNFRSF8, CCL18, CD163, TGFα.

In particular, using the six additional biomarkers is advantageous in that it allows higher sensitivity and higher specificity as can be observed on Table 2a but renders the test more difficult to implement.

The choice of the number of additional biomarkers can be based on a financial cost criterion and/or a test duration criterion and/or on a required sample column criterion and/or on a technological criterion, including for example the maximum number of biomarkers that can be implemented on the same test to date depending among others on the cost, on the test duration and on the sample volume.

In particular, the inventors have shown that the combination of the amounts of the of CCL2 and CEACAM1 biomarkers indicates that the individual is more likely to give birth within N days after test day TD than not, N being positive and optionally ranging from 7 days to 14 days, as shown in Table 3.

Table 3 shows in particular that the sensitivity (respectively the specificity) of the combination consisting of CCL2-CEACAM1, as well as the combination consisting of CCL2-CEACAM1 and MCIL1RL1, CD14, TNFRSF8, CCL18, CD163 and TGFα, decreases (respectively increases) when N increases from 7 days to 14 days, the test having very good performances in the whole range.

Note than 7 days before birth is an optimal window for antenatal corticosteroid therapy in the case of preterm birth, as shown in Melamed N, Asztalos E. Antenatal betamethasone regimen for women at risk of preterm birth. Lancet. 2022 Aug 20;400(10352):541-543, rendering such a test particularly interesting.

Further, a simultaneous increase of the amount of each of the biomarkers the CCL2-CEACAM1 combination of biomarkers may indicate that the individual is more likely to give birth within N days after test day TD than not, N being positive and optionally ranging from 7 days to 14 days. Such an increase can be observed is the test has already been performed once before test day TD, at a so-called "previous test day" PTD.

The result of the test according to the invention can be based on an algorithm or a mathematical function of the measured amounts of each of the biomarkers of the combination of biomarkers.

In particular, the inventors have shown that when simultaneously the amount of the biomarker CCL2 is higher than a threshold of 22 pg/ml in the sample of an individual and the amount of the biomarker CEACAM1 is higher than a threshold 12 ng/ml in the sample of an individual, the individual is more likely to give birth within N days of test day TD than not, N being positive and optionally ranging from 7 days to 14 days.

In one embodiment, the combination of biomarkers of the invention comprises or consists of CCL2 and CEACAM1 as biomarkers for upcoming delivery.

In one embodiment, the combination of biomarkers of the invention may further comprise one or more additional biomarkers for upcoming delivery selected from the group comprising IL1RL1, CD14, TNFRSF8, CCL18, CD163, TGFα or any combination thereof.

The inventors have in particular shown that the amount of one or more additional biomarkers selected from the group comprising IL1RL1, CD14, TNFRSF8, CCL18, CD163, TGFα, in combination with the amount of both CCL2 and CEACAM1 may indicate upcoming delivery.

As used herein, "IL1RL1" may refer to the interleukin 1 receptor like 1 gene or to the interleukin-1 receptor-like 1 protein. According to the present invention, "IL1RL1" or "IL33R", or "growth stimulation-expressed" or "homolog of mouse growth stimulation-expressed" or "interleukin 1 receptor-related protein" can be used interchangeably.

As used herein, "CD14" may refer to the CD14 molecule gene or to the monocyte differentiation antigen CD14 protein. According to the present invention, "CD14" or "myeloid cell-specific leucine-rich glycoprotein" can be used interchangeably.

As used herein, "TNFRSF8" may refer to the TNF receptor superfamily member 8 gene or to the tumor necrosis factor receptor superfamily member 8 protein. According to the present invention, "TNFRSF8" or "CD30" or "CD30L receptor" or "Ki-1 antigen" or "cytokine receptor CD30" or "lymphocyte activation antigen CD30" can be used interchangeably.

As used herein, "CCL18" may refer to the C-C motif chemokine ligand 18 gene or to the C-C motif chemokine 18 protein. According to the present invention, "CCL18" or "PARC" or "CC chemokine PARC" or "CC chemokine ligand 18" or "alternative macrophage activation-associated CC chemokine 1" or "chemokine (C-C motif) ligand 18 (pulmonary and activation-regulated)" or "chemokine (C-C) dendritic" or "dendritic cell chemokine 1" or "macrophage inflammatory protein 4" or "pulmonary and activation-regulated chemokine" or "small inducible cytokine A18" or "small inducible cytokine subfamily A (Cys-Cys) member 18 pulmonary and activation-regulated" or "small inducible cytokine subfamily A (Cys-Cys) member 18 pulmonary and activation-regulated" can be used interchangeably.

As used herein, "CD163" may refer to the CD163 molecule gene or to the scavenger receptor cysteine-rich type 1 protein M130. According to the present invention, "CD163" or "hemoglobin scavenger receptor" or "macrophage-associated antigen" can be used interchangeably.

As used herein, TGFα may refer to the transforming growth factor alpha protein or the TGFα gene.

In one embodiment, the combination of biomarkers of the invention comprises or consists of:
a) CCL2 and CEACAM1
b) and one additional biomarker chosen in the list: IL1RL1, CD14, TNFRSF8, CCL18, CD163, TGFα.

In one embodiment, the combination of biomarkers of the invention comprises or consists of :
a) CCL2 and CEACAM1
b) and one additional combination of biomarkers chosen in the list IL1RL1 and CD14; IL1RL1 and TNFRSF8; IL1RL1 and CCL18; IL1RL1 and CD163, IL1RL1 and TGFα; CD14 and TNFRSF8; CD14 and CCL18; CD14 and CD163; CD14 and TGFα ;TNFRSF8 and CCL18; TNFRSF8 and CD163; TNFRSF8 and TGFα; CCL18 and CD163; CCL18 and TGFα; CD163 and TGFα

In one embodiment, the combination of biomarkers of the invention comprises or consists of :
a) CCL2 and CEACAM1
b) and one additional combination of two biomarkers chosen in the list: IL1RL1 and CD14; IL1RL1 and TNFRSF8; IL1RL1 and CCL18; IL1RL1 and CD163, IL1RL1 and TGFα; CD14 and TNFRSF8; CD14 and CCL18; CD14 and CD163; CD14 and TGFα ;TNFRSF8 and CCL18; TNFRSF8 and CD163; TNFRSF8 and TGFα; CCL18 and CD163; CCL18 and TGFα; CD163 and TGFα.

In one embodiment, the combination of biomarkers of the invention comprises or consists of :
a) CCL2 and CEACAM1
b) and one additional combination of three biomarkers chosen in the list:
   IL1RL1 and CD14 and TNFRSF8; IL1RL1 and CD14 and CCL18; IL1RL1 and CD14 and CD163; IL1RL1 and CD14 and TGFα; IL1RL1 and TNFRSF8 and CCL18; IL1RL1 and TNFRSF8 and CD163; IL1RL1 and TNFRSF8 and TGFα; IL1RL1 and CCL18 and CD163; IL1RL1 and CCL18 and TGFα ; IL1RL1 and CD163 and TGFα; CD14 and TNFRSF8 and CCL18; CD14 and TNFRSF8 and CD163; CD14 and TNFRSF8 and TGFα ; CD14 and CCL18 and CD163; CD14 and CCL18 and TGFα ; CD14 and CD163 and TGFα ; TNFRSF8 and CCL18 and CD163; TNFRSF8 and CCL18 and TGFα ; CCL18 and CD163 and TGFα .

In one embodiment, the combination of biomarkers of the invention comprises or consists of :
a) CCL2 and CEACAM1
b) and one additional combination of four biomarkers chosen in the list:
   IL1RL1 and CD14 and TNFRSF8 and CCL18 ; IL1RL1 and CD14 and TNFRSF8 and CD163 ; IL1RL1 and CD14 and TNFRSF8 TGFα; IL1RL1 and CD14 and CD163 and CCL18 ; IL1RL1 and CD14 and CD163 and TGFα ; IL1RL1 and CD14 and CCL18 and TGFα ; IL1RL1 and TNFRSF8 and CCL18 and CD163 ; IL1RL1 and TNFRSF8 and CCL18 and TGFα ; IL1RL1 and CD163 and CCL18 and TGFα ; IL1RL1 and TNFRSF8 and CD163 and TGFα; CD14 and TNFRSF8 and CCL18 and CD163 ; CD14 and TNFRSF8 and CCL18 and TGFα ; CD14 and TNFRSF8 and TGFα and CD163 ; CD14 and CCL18 and CD163 and TGFα; CD14 and TNFRSF8 and CCL18 and TGFα.

In one embodiment, the combination of biomarkers of the invention comprises or consists of:
a) CCL2 and CEACAM1
b) and one additional combination of five biomarkers chosen in the list : IL1RL1 and CD14 and TNFRSF8 and CCL18 and CD163; IL1RL1 and CD14 and TNFRSF8 and CCL18 and TGFα; IL1RL1 and TNFRSF8 and CCL18 and CD163 and TGFα ; CD14 and TNFRSF8 and CCL18 and CD163 and TGFα; IL1RL1 and CD14 and TNFRSF8 and CD163 and TGFα; IL1RL1 and CD14 and TNFRSF8 and CCL18 and TGFα.

In one embodiment, the combination of biomarkers of the invention comprises CCL2, CEACAM1, IL1RL1, CD14, TNFRSF8, CCL18, CD163 and TGFα.

Advantageously, the increased amount of a combination of biomarkers comprising CCL2, CEACAM1, IL1RL1, CD14, TNFRSF8, CCL18, CD163, TGFα may indicate that the individual is at risk of imminent birth or preterm delivery.

In one embodiment, the combination of biomarkers of the invention solely comprises CCL2, CEACAM1, IL1RL1, CD14, TNFRSF8, CCL18, CD163 and TGFα as biomarkers for upcoming delivery.

In one advantageous embodiment, the combination of biomarkers contains neither COL1A1 nor CCN5.

In a particular embodiment, the combination of biomarkers for preterm delivery has a sensitivity higher than 90% and a specificity higher than 75% for predicting upcoming birth within N = 7 days of test day when tested with cohort 2 of example 1, this test being considered in the present application as the reference cohort for assessing the sensitivity and the specificity of a combination of biomarkers of upcoming birth.

In a particular embodiment, the combination of biomarkers for preterm delivery has a sensitivity higher than 90% and a specificity higher than 75% for predicting upcoming birth within N = 8 days and/or within N= 9 days and/or within N = 10 days of test day when tested with cohort 2 of example 1, this test being considered in the present application as the reference cohort for assessing the sensitivity and the specificity of a combination of biomarkers of upcoming birth.

In a particular embodiment, the combination of biomarkers for preterm delivery has a sensitivity higher than 83% and a specificity higher than 83% for predicting upcoming birth within N = 11 days and/or within N = 12 days and/or within N=13 days and/or within = 14 days of test day when tested with cohort 2 of example 1, this test being considered in the present application as the reference cohort for assessing the sensitivity and the specificity of a combination of biomarkers of upcoming birth.

The present invention also provides the use of a combination of biomarkers comprising or consisting of CCL2 and CEACAM1 for predicting upcoming delivery:
- either for predicting preterm birth if the test is performed at a test day TD at least N days before 37 weeks minus 1 day, N being positive and optionally ranging from 7 to 14 ;
- or for predicting spontaneous labor at term if the test is performed at a test day TD between N days before 37 weeks and N days before 41 weeks and 6 days, N being positive and optionally ranging from 7 to 14;
- or for predicting upcoming post term labor if the test is performed at a test day TD strictly after N days before 41 weeks and 6 days.

In a particular embodiment, the combination of biomarkers for upcoming delivery invention that is made used of further comprises one or more additional biomarkers selected from the group comprising IL1RL1, CD14, TNFRSF8, CCL18, CD163 and TGFα or any combination thereof.

For example, at least two, three, four, five or six different biomarkers may be used according to the present invention. All the combination of one or more of the six additional biomarkers {IL1RL1, CD14, TNFRSF8, CCL18, CD163, TGFα} which have been described above may be used of according to the present invention.

In one embodiment, the combination of biomarkers for upcoming delivery invention that is made used of comprises neither COL1A1 nor CCN5.

According to the present invention, the combination of biomarkers of the invention that is made used of may be a combination of one or more protein selected from the group comprising CCL2, CEACAM1, IL1RL1, CD14, TNFRSF8, CCL18, CD163 and TGFα and/or one or more nucleic acid encoding for one of said proteins.

The present invention also provides methods for predicting upcoming delivery of a pregnant individual
- either for predicting preterm delivery if the test is performed at a test day TD at least N days before 37 weeks minus 1 day, N being positive and optionally ranging from 7 to 14 ;
- or for predicting spontaneous labor at term if the test is performed at a test day TD between N days before 37 weeks and N days before 41 weeks and 6 days, N being positive and optionally ranging from 7 to 14;
- or for predicting upcoming post term labor if the test is performed at a test day TD strictly after N days before 41 weeks and 6 days.

Any of the combination of biomarkers according to the invention can be implemented. Such combination of biomarkers may allow the diagnostic distinction between preterm delivery and/or upcoming birth and other conditions that exhibit similar symptoms. Such combination of biomarkers may also allow the prediction of preterm delivery and/or the prediction of imminent spontaneous delivery either at term or post term.

In particular, early identification of individuals at greater risk for preterm delivery and/or imminent birth would be of considerable value, as such subjects could be more closely monitored.

Advantageously, the present invention provides a method for prognosing and/or for diagnosing upcoming delivery of a pregnant individual within N days after test day TD, N being positive and optionally ranging from 7 to 14, the method comprising:
a) determining the presence and/or amount of a combination of biomarkers for upcoming delivery in a sample obtained from said pregnant individual, and
b) comparing the presence and/or amount of said combination of biomarkers obtained from step a) to the presence and/or amount of the same combination of biomarkers in a control sample c) determining based on this comparison if the pregnant individual is more likely than not to deliver within N days after test day TD,
wherein said combination of biomarkers comprises CCL2 and CEACAM1.

As used herein, the term "diagnostic" or "diagnosing" refers to the procedure through which the presence of absence of a condition is identified in an individual and, the term "prognosis" or "prognosing" refers to the procedure through which a prediction is made of the events that will occur in the development or course of a condition.

In a particular embodiment, the condition is the preterm delivery.

In one particular embodiment, the condition is upcoming birth at term.

In another embodiment, the condition is upcoming birth post term.

Advantageously, the inventors have shown that the method according to the invention makes it possible to exclude upcoming delivery within N days after test day and thus:
- either exclude the risk of preterm delivery within these N days, when none or only one of the biomarkers are detected. In other words, the method according to the invention makes it possible to conclude with increased reliability on the absence of threat of preterm delivery, thus making it possible to avoid unnecessary hospitalizations.
- or exclude imminent birth and thus exclude the risk of preterm delivery, when none or only one of the biomarkers are detected. In other words, the method according to the invention makes it possible to conclude with increased reliability on the absence of threat of imminent birth within up to N days in the term period, thus making it possible for example to avoid too early hospitalizations, to better organize maternity staffs and pregnant women way of life near pregnancy term.
- or obtain information in order to decide of labor induction and/or caesarean section.

In one aspect, prior to step a), the method may further comprise a step of obtaining a sample from an individual. Advantageously, the sample used in step a) is a sample of vaginal fluid, advantageously a sample of vaginal secretion. The sample that is taken from the individual may vary, but the sampling preferably is minimally invasive and is easily performed by conventional techniques.

Advantageously, methods disclosed herein can be used to determine the risk or likelihood of upcoming delivery in asymptomatic and/or symptomatic pregnant individuals.

Testing of individual using the methods described herein may occur at any time during pregnancy, when the combination of biomarkers indicative of preterm delivery and/or imminent birth is quantifiable in the individual.

In one embodiment, the combination of biomarkers may be tested at 20 weeks of gestation, advantageously at 21 weeks of gestation, advantageously at 22 weeks of gestation, advantageously at 23 weeks of gestation, advantageously at 24 weeks of gestation, advantageously at 25 weeks of gestation, advantageously at 26 weeks of gestation, advantageously at 27 weeks of gestation, advantageously at 28 weeks of gestation, advantageously at 29 weeks of gestation, advantageously at 30 weeks of gestation, advantageously at 31 weeks of gestation, advantageously at 32 weeks of gestation, advantageously at 33 weeks of gestation, advantageously at 34 weeks of gestation, advantageously at 35 weeks of gestation, advantageously at 36 weeks of gestation, advantageously at 37 weeks of gestation, advantageously at 38 weeks of gestation advantageously at 39 weeks of gestation, advantageously at 40 weeks of gestation, advantageously at 41 weeks of gestation, advantageously at 42 weeks of gestation.

In another embodiment, the combination of biomarkers may be tested at least one, and optionally repeatedly, from about 20 weeks to 42 weeks of gestation.

In a particular embodiment, the combination of biomarkers may be tested from 24 weeks to N days before 36 weeks and 6 days of gestation, or from 24 weeks to 34 weeks of gestation. Advantageously, the combination of biomarkers may be tested at from about 28 weeks to about 32 weeks of gestation.

In a particular embodiment, the combination of biomarkers may be tested from N days before from about 37 weeks to N days before 41 weeks and 6 days of gestation.

In a particular embodiment, the combination of biomarkers may be tested after N days before 41 weeks and 6 days of gestation.

It should be noted that these ranges should not be seen as limiting, as such testing may be performed at any point during pregnancy. Rather these ranges are provided to demonstrate periods of the gestational cycle, where such testing is most likely to occur in a majority of individuals.

Next, the method comprises determining the presence and/or amount of a combination of biomarkers for preterm delivery in a sample obtained from said individual, wherein said combination of biomarkers comprises CCL2 and CEACAM1.

Variation of the amount of the biomarker, and in particular an increase in this amount, as compared to a control or reference amount, may indicate that the individual is at risk of preterm delivery and/or imminent birth.

In this case, steps a) and b) of the method are performed at least two time points, including:
- at least one time point being before test day,
- and a time point at test day.

Presence or absence of the biomarker, as compared to a control or reference, may indicate upcoming delivery.

Such methods involve determining the presence and/or the amount of CCL2 and CEACAM1 in a sample obtained from the individual being tested.

In one embodiment, the combination of biomarkers used in step a) may further comprise, one or more additional biomarkers for preterm delivery selected from the group comprising IL1RL1, CD14, TNFRSF8, CCL18, CD163 and TGFα or any combination thereof, which is to say any combination of one or more of the six additional biomarkers {IL1RL1, CD14, TNFRSF8, CCL18, CD163, TGFα} which have been described above.

In a particular embodiment, the one or more additional biomarkers for upcoming birth consists of any one combination of one or more of the six additional biomarkers {IL1RL1, CD14, TNFRSF8, CCL18, CD163, TGFα} which have been described above.

In a particular embodiment, the method of the invention makes it possible to predict preterm delivery and thus a high risk of preterm delivery, advantageously a high risk of preterm delivery and/or imminent birth if the biomarkers CCL2 and CEACAM1 and one or more of the six additional biomarkers {IL1RL1, CD14, TNFRSF8, CCL18, CD163, TGFα} are present in the sample obtained from an individual.

Variation of the amount of the one or more additional biomarker in addition to CCL2 and CEACAM1, as compared to a control or reference amount, may indicate that the individual is at risk of preterm delivery and/or indicate upcoming birth.

Presence or absence of the one or more additional biomarker in addition to CCL2 and CEACAM1, as compared to a control or reference, may indicate that the individual is at risk of preterm delivery and/or indicate upcoming birth.

Such methods involve determining at step a) the presence and/or the amount of IL1RL1 and/or CD14 and/or TNFRSF8 and/or CCL18 and/or CD163 and/or TGFα in a sample obtained from the individual being tested.

In a particular embodiment, the combination of additional biomarkers for upcoming delivery used in step a) comprises, optionally consists of, IL1RL1, CD14, TNFRSF8, CCL18, CD163, TGFα.

In a particular embodiment, the combination of biomarkers for upcoming delivery used in step a) comprises neither COL1A1 nor CCN5.

Measurements of biomarker may include, for example, measurements that indicate the presence, amount, expression level, or any other value associated with a biomarker. The biomarkers of the invention may be detected at the nucleic acid or protein level. Thus, the biomarkers of the invention may be nucleic acid encoding for a protein such as DNA, RNA or a protein and may be detected using any appropriate technique. The presence and/or amount of the combination of biomarkers of the invention may be measured directly or indirectly. Any appropriate agent may be used to determine the presence and/or amount of the one or more biomarker of the invention. For example, the presence and/or amount of the combination of biomarkers of the invention may be determined using an agent selected from peptides and peptidomimetics, antibodies, small molecules and oligonucleotides such as single-stranded DNA or RNA molecules, as described herein. The relative presence and/or amount of the combination of biomarkers of the invention relative to a control sample may be determined using any appropriate technique. Suitable standard techniques are known in the art.

For example, when the biomarkers of the combination are detected at the nucleic acid level this may be carried out using: (i) biomarker-specific oligonucleotide DNA or RNA or any other nucleic acid derivative probes bound to a solid surface; (ii) purified RNA (labelled by any method, for example using reverse transcription and amplification) hybridized to probes; (iii) purified RNA hybridized to probes and a second probe (labelled by any method ) hybridized to the purified RNA; (iv) RT-PCR using any primer/probe combination or inter-chelating fluorescent label; (v) end-point PCR; (vi) digital PCR; (vii) sequencing; (viii) array cards (RT-PCR); (ix) lateral flow devices/methodology; and/or (x) digital microfluidics.

For example, when the biomarkers of the combination are detected at the protein acid level this may be carried out using: (i) biomarker-specific primary antibodies or antibody fragments bound to a solid surface; (ii) secondary biomarker-specific antibodies or antibody fragments used to detect biomarker antigen bound to primary antibody (labelled using any method); (iii) biomarker-specific primary aptamers bound to a solid surface; (iv) secondary biomarker-specific aptamer used to detect biomarker antigen bound to primary aptamer (labelled using any method); (v) any antibody derivative i.e. phage display etc. used as above; (vi) lateral flow devices/methodology; (vii) chromatography; (viii) mass spectrometry; (ix) nuclear magnetic resonance (NMR); (x) protein gels/transfers to filter; and/or (xi) immunoprecipitation.

The presence and/or amount of the biomarkers of the combination of the invention may be determined by quantitative and/or qualitative analysis. The amount of the biomarkers of the combination of the invention encompasses the mass of the biomarkers of the combination, the molar amount of the biomarkers of the combination, the concentration of the biomarkers of the combination and the molarity of the biomarkers of the combination. This amount may be given in any appropriate units. For example, the concentration of the biomarkers of the combination may be given in pg/ml, ng/ml, µg/ml or mg/ml.

Any agent for the detection of or for the determination of the amount of the biomarkers of the combination of the invention may be used to determine the presence of and/or amount of the biomarkers of the combination. Similarly, any method that allows for the diagnosing and/or prognosing of the biomarkers of the combination, the quantification, or relative quantification of the biomarkers of the combination may be used.

Agents for the detection of or for the determination of the amount of one biomarker present in the combination or several biomarkers of the combination may be used to determine the amount of the one biomarker present in the combination or several biomarkers of the combination in a sample obtained from the individual. Such agents typically bind to one biomarker present in the combination or several biomarkers of the combination. Such agents may bind specifically to one biomarker present in the combination or several biomarkers of the combination. The agent for the detection of or for the determination of the amount of the biomarkers of the combination may be an antibody or other binding agent specific for one biomarker present in the combination or several biomarkers of the combination.

By specific, it will be understood that the agent or antibody binds to the molecule of interest, in this case one or more biomarkers of the combination, with no significant cross-reactivity to any other molecule, particularly any other protein. Cross-reactivity may be assessed by any suitable method. Cross-reactivity of an agent or antibody for one or more biomarkers of the combination with a molecule other than one or more biomarkers of the combination may be considered significant if the agent or antibody binds to the other molecule at least 5%, 10%, 15%, 20%, 25%, 30%, 35%, 40%, 45%, 50%, 55%, 60%, 65%, 70%, 75%, 80%, 85%, 90% or 100% as strongly as it binds to one or more biomarkers of the combination. An agent or antibody that is specific for one or more biomarkers of the combination may bind to another molecule at less than 90%, 85%, 80%, 75%, 70%, 65%, 60%, 55%, 50%, 45%, 40%, 35%, 30%, 25% or 20%) the strength that it binds to one or more biomarkers of the combination. Advantageously, the agent or antibody binds to the other molecule at less than 20%, less than 15%, less than 10% or less than 5%), less than 2% or less than 1% the strength that it binds to one or more biomarkers of the combination.

In one embodiment, in step a), the presence and/or amount of the CCL2 and CEACAM1 biomarkers is determined using an antibody specific for said CCL2 and CEACAM1 biomarkers. Advantageously, when the combination of biomarkers of upcoming delivery comprises one or more additional biomarkers in combination with CCL2 and CEACAM1 biomarkers, the presence and/or amount of the one or more additional biomarkers is determined using an antibody specific for said one or more additional biomarkers.

As described herein, the presence and/or amount of the combination of biomarkers may be determined immunologically by reacting antibodies, or functional fragments thereof, specific to the biomarkers. A functional fragment of an antibody is a portion of an antibody that retains at least some ability to bind to the antigen to which the complete antibody binds. The fragments, which include, but are not limited to, scFv fragments, Fab fragments, F(ab) fragments and F(ab)2 fragments, can be recombinantly produced or enzymatically produced. Specific binding molecules other than antibodies, such as aptamers, may be used to bind the biomarkers.

The antibody may be monoclonal or polyclonal. The antibody may be produced by any suitable method known in the art. For example, polyclonal antibodies may be obtained by immunizing a mammal, typically a rabbit or a mouse, with the one or more biomarker under suitable conditions and isolating antibody molecules from, for example, the serum of said mammal. Monoclonal antibodies may be obtained by hybridoma or recombinant methods. Hybridoma methods may involve immunizing a mammal, typically a rabbit or a mouse, with the one or more biomarker under suitable conditions, then harvesting the spleen cells of said mammal and fusing them with myeloma cells. The mixture of fused cells is then diluted and clones are grown from single parent cells. The antibodies secreted by the different clones are then tested for their ability to bind to the one or more biomarker, and the most productive and stable clone is then grown in culture medium to a high volume. The secreted antibody is collected and purified.

Recombinant methods may involve the cloning into phage or yeast of different immunoglobulin gene segments to create libraries of antibodies with slightly different amino acid sequences. Those sequences which give rise to antibodies which bind to the one or more biomarkers may be selected, and the sequences cloned into, for example, a bacterial cell line, for production. Typically, the antibody is a mammalian antibody, such as a primate, human, rodent (e.g., mouse or rat), rabbit, ovine, porcine, equine or camel antibody. The antibody may be a camelid antibody or shark antibody. The antibody may be a nanobody. The antibody can be any class or isotype of antibody, for example IgM, but is preferably IgG. The antibody may be a humanized antibody.

The antibody or fragment may be associated with other moieties, such as linkers which may be used to join together 2 or more fragments or antibodies. Such linkers may be chemical linkers or can be present in the form of a fusion protein with the fragment or whole antibody. The linkers may thus be used to join together whole antibodies or fragments which have the same or different binding specificities, e.g., that can bind the same or different polymorphisms. The antibody may be a bispecific antibody which is able to bind to two different antigens, typically any two of the polymorphisms mentioned herein. The antibody may be a 'diabody' formed by joining two variable domains back-to-back. In the case where the antibodies used in the method are present in any of the above forms which have different antigen binding sites of different specificities then these different specificities are typically to polymorphisms at different positions or on different proteins. In one embodiment the antibody is a chimeric antibody comprising sequence from different natural antibodies, for example a humanized antibody.

Methods to assess an amount of the biomarkers of the combination may involve contacting a sample with an agent or antibody capable of binding specifically to the biomarkers of the combination. Such methods may include dipstick assays and Enzyme-linked Immunosorbent Assay (ELISA), or similar assays, such as those using a lateral flow device. Other immunoassay types may also be used to assess the one or more biomarker amounts. Typically, dipsticks comprise one or more antibodies or proteins that specifically bind to the biomarkers of the combination. If more than one antibody is present, the antibodies preferably have different non-overlapping determinants such that they may bind to the biomarkers of the combination simultaneously.

ELISA is a heterogeneous, solid phase assay that requires the separation of reagents. ELISA is typically carried out using the sandwich technique or the competitive technique. The sandwich technique requires two antibodies. The first specifically binds one or more biomarkers of the combination and is bound to a solid support. The second antibody is bound to a marker, typically an enzyme conjugate. A substrate for the enzyme is used to quantify biomarkers of the combination -antibody complex and hence the amount of the biomarkers of the combination in a sample. The antigen competitive inhibition assay also typically requires that the biomarkers of the combination-specific antibody bound to a support. A biomarker-enzyme conjugate is added to the sample containing one or more biomarkers of the combination to be assayed. Competitive inhibition between the biomarker-enzyme conjugate and unlabeled biomarker allows quantification of the amount of the biomarkers of the combination in a sample. The solid supports for ELISA reactions preferably contain wells.

Multiplex approach such as antibodies on Luminex beads (for example of the multiplex bio-rad), the approach singleplex or multiplex by chemiluminescence of the company MesoScale Discovery (MSD), or antibodies on flat surface ("Antibodies-Arrays") (for example the approach proposed by the company MesoScale Discovery) may also be used for determining the presence and/or amount of the combination of biomarkers of the invention.

Antibodies capable of binding specifically to the biomarkers of the combination may be used in methods of immunofluorescence to detect the presence of the biomarkers of the combination and hence in methods of diagnosing and/or prognosing the risk of preterm delivery and/or imminent birth according to the present invention.

The present invention may also employ methods of determining the amount of the biomarkers of the combination that do not comprise antibodies. High Performance Liquid Chromatography (HPLC) separation and fluorescence detection is preferably used as a method of determining the amount of the biomarkers of the combination.

Other methods of determining the amount the biomarkers of the combination that do not comprise antibodies include mass spectrometry. Mass spectrometric methods may include, for example, matrix-assisted laser desorption/ionization mass spectrometry (MALDI MS), surface-enhanced laser desorption/ionization mass spectrometry (SELDI MS), time of flight mass spectrometry (TOF MS) and liquid chromatography mass spectrometry (LC MS). In that case, prior to the measurements, a fixed amount of a least one substance serving as the at least on internal standard is added to the original sample and the intensity of its peak is also measured. The concentration of the target in the sample can be calculated from the ratio of peak intensity of the target to the peak intensity of the at least one internal standard.

A separation method may be used to determine the presence and/or amount of the biomarkers of the combination, such that only a subset of biomarkers within the sample is analyzed. For example, the biomarkers that are analyzed in a sample may consist of mRNA species from a cellular extract, which has been fractionated to obtain only the nucleic acid biomarkers within the sample, or the biomarkers may consist of a fraction of the total complement of proteins within the sample, which have been fractionated by chromatographic techniques. One or more, two or more, three or more, four or more, or five or more separation methods may be used according to the present invention.

Determination of the presence and/or amount of the biomarkers of the combination may be carried out without employing a separation method. For example, a biological sample may be interrogated with a labelled compound that forms a specific complex with a biomarker in the sample, where the intensity of the label in the specific complex is a measurable characteristic of the biomarker. A suitable compound for forming such a specific complex is a labelled antibody. A biomarker may be measured using an antibody with an amplifiable nucleic acid as a label. The nucleic acid label may become amplifiable when two antibodies, each conjugated to one strand of a nucleic acid label, interact with the biomarker, such that the two nucleic acid strands form an amplifiable nucleic acid.

The presence and/or amount of the biomarkers of the combination may be derived from an assay, such as an array, of nucleic acids, where the biomarkers are the nucleic acids or complements thereof. For example, the biomarkers may be nucleic acids. The presence and/or amount of the biomarkers of the combination may be obtained using a method selected from nuclear magnetic resonance, nucleic acid arrays, dot blotting, slot blotting, reverse transcription amplification, Northern analysis and quantitative real-time PCR (qPCR).

The determination of the presence and/or amount of the biomarkers of the combination may be generated by the use of one or more separation methods. For example, suitable separation methods may include a mass spectrometry method, such as electrospray ionization mass spectrometry (ESI-MS), ESI-MS/MS, ESI- MS/(MS)n (n is an integer greater than zero), matrix-assisted laser desorption ionization time- of-flight mass spectrometry (MALDI-TOF-MS), surface-enhanced laser desorption/ionization time-of-flight mass spectrometry (SELDI-TOF-MS), desorption/ionization on silicon (DIOS), secondary ion mass spectrometry (SLMS), quadrupole time-of-flight (Q-TOF), atmospheric pressure chemical ionization mass spectrometry (APCI-MS), APCI-MS/MS, APCI-(MS)n, atmospheric pressure photoionization mass spectrometry (APPI-MS), APPI-MS/MS, and APPI-(MS)n. Other mass spectrometry methods may include, inter alia, quadrupole, Fourier transform mass spectrometry (FTMS) and ion trap. Other suitable separation methods may include chemical extraction partitioning, column chromatography, ion exchange chromatography, hydrophobic (reverse phase) liquid chromatography, isoelectric focusing, one-dimensional polyacrylamide gel electrophoresis (PAGE), two-dimensional polyacrylamide gel electrophoresis (2D-PAGE) or other chromatography, such as thin-layer, gas or liquid chromatography, or any combination thereof. The sample may be fractionated prior to application of the separation method.

The determination of the presence and/or amount of the biomarkers of the combination may be generated by methods that do not require physical separation of the biomarkers themselves. For example, nuclear magnetic resonance (NMR) spectroscopy may be used to resolve a profile of biomarkers from a complex mixture of molecules.

In another embodiment, the total mRNA from a cellular extract of the individual is assayed, and the various mRNA species that are obtained from the sample are used as biomarkers. Biomarker may be obtained, for example, by hybridizing these mRNAs to an array of probes, which may comprise oligonucleotides or cDNAs, using standard methods known in the art. Alternatively, the mRNAs may be subjected to gel electrophoresis or blotting methods such as dot blots, slot blots or Northern analysis, all of which are known in the art. mRNA profiles also may be obtained by reverse transcription followed by amplification and detection of the resulting cDNAs. In another embodiment, the profile may be obtained by using a combination of methods, such as a nucleic acid array combined with mass spectroscopy.

Different methods have different advantages and may be preferred depending on numerous factors, such as the particular circumstances of the individuals to be tested and/or the availability of reagents/equipment in the diagnostics laboratory. For example, qPCR using probe/quencher hydrolysis probes as described herein is highly specific and stringent. As another example, microarray analysis can resolve subtle differences in expression of transcript variants, which may be important in disease pathology and diagnosis.

Any appropriate detection means can be used to detect or quantify the biomarkers of the combination of the invention, as described herein.

Typically, when the biomarkers of the combination of the invention are a nucleic acid, the presence of the biomarkers of the combination may be detected, and/or the amount of the biomarkers of the combination determined using an oligonucleotide probe.

In one embodiment, in step a), the presence and/or amount of the CCL2 and CEACAM1 biomarkers is determined using an oligonucleotide probe specific for said CCL2 and CEACAM1 biomarkers. Advantageously, when the combination of biomarkers for upcoming delivery comprising CCL2 and CEACAM1 biomarkers comprises one or more additional biomarkers, the presence and/or amount of the one or more additional biomarkers is determined using an oligonucleotide probe specific for said one or more additional biomarkers.

An oligonucleotide probe of the invention may have at least 80% sequence identity to the biomarkers of the combination of the invention, or a target region within said biomarkers, measured over any appropriate length of sequence. Typically, the % sequence identity is determined over a length of contiguous nucleic acid residues. An oligonucleotide probe of the invention may, for example, have at least 80% sequence identity to the biomarkers of the combination of the invention, or target region thereof, measured over at least 10, at least 20, at least 30, at least 40, at least 50, at least 60, at least 70, at least 80, at least 90, or more nucleic acid residues, up to the oligonucleotide probe having at least 80%> sequence identity with the biomarkers of the combination of the invention, or target region thereof, over the entire length of the oligonucleotide probe.

An oligonucleotide probe of the invention may be complementary to the one or more nucleic acid biomarker of the invention, or a target region thereof. Typically, the oligonucleotide probe of the invention is complementary over a length of contiguous nucleic acid residues. An oligonucleotide probe of the invention may, for example, be complementary to the biomarkers of the combination of the invention, or target region thereof, measured over at least 10, at least 20, at least 30, at least 40, at least 50, at least 60, at least 70, at least 80, at least 90, or more nucleic acid residues, up to the oligonucleotide probe having being complementary to the one or more biomarker of the invention, or target region thereof, over the entire length of the oligonucleotide probe.

Any of a variety of sequence alignment methods can be used to determine percent identity, including, without limitation, global methods, local methods and hybrid methods, such as, e.g., segment approach methods. Protocols to determine percent identity are routine procedures within the scope of one skilled in the art. Global methods align sequences from the beginning to the end of the molecule and determine the best alignment by adding up scores of individual residue pairs and by imposing gap penalties. The probes of the invention are typically designed to hybridize to their target nucleic acid sequence present in the biomarkers of the combination of the invention.

Typically, probes of the invention are oligonucleotides having sequence identity with a region of the biomarkers of the combination of the invention as disclosed herein. One or more probes may be immobilized on a solid support, and used to interrogate mRNA obtained from a test sample. If the mRNA from the test sample contains the one or more biomarker targeted by the immobilized probe, it will bind to the probe, and may then be detected. The biomarkers of the invention may also be detected using PCR, such as real time PCR. Any oligonucleotide with the appropriate level of sequence identity with the biomarkers of the combination of the invention, or with one or more target sequences within said biomarkers of the combination of the invention may be used as a probe as described herein. Any oligonucleotide with the appropriate level of complementarity with the one or more biomarker of the invention, or with one or more target sequences within said biomarkers of the combination of the invention may be used as a probe as described herein.

Next, the method comprises a step b) consisting of comparing the presence and/or amount of said combination of biomarkers obtained from step a) to the presence and/or amount of the same combination of biomarkers in a control sample, to evaluate a likelihood of upcoming birth.

As used herein, "comparing" or "comparison" includes any means to discern at least one difference in the presence and/or amount of the biomarkers of the combination in the individual and the control sample. Thus, a comparison may include a visual inspection of chromatographic spectra, and a comparison may include arithmetical or statistical comparisons of values assigned to the features of the profiles. Such statistical comparisons include, but are not limited to, applying a decision rule. The comparison can confirm the presence or absence of preterm delivery and/or imminent birth, and thus to prognose or diagnose upcoming delivery.

Typically, the control sample corresponds to a sample of vaginal fluid, advantageously a sample of vaginal secretion obtained from individual that did not experience preterm delivery and/or imminent birth. In other words, the control sample corresponds to a sample of vaginal fluid obtained from individual, who had normal birth, also named control population.

Advantageously, in the control sample, the amount of the biomarkers is as follows:
- amount of CCL2 is less or equal to 22 pg/mL, and
- amount of CEACAM1 is less or equal to 12 ng/mL.

In another embodiment, the method may further comprise comparing the presence and/or amount of the combination of biomarkers obtained from step a) to the presence and/or amount of the same combination of biomarkers in a control sample; and classifying the individual as belonging to or not belonging to the control population, wherein the comparison determines in increased risk of upcoming birth with N days after test day TD and wherein the combination of biomarkers comprises CCL2 and CEACAM1.

In another embodiment, the method may further comprise comparing the presence and/or amount of the combination of biomarkers obtained from step a) to the presence and/or amount of the same combination of biomarkers in a control sample; and classifying the individual as belonging to or not belonging to the control population, wherein the comparison determines in increased risk for preterm delivery and/or imminent birth, and wherein the combination of biomarkers comprises CCL2 and CEACAM1 and one or more additional biomarkers selected among the group comprising IL1RL1, CD14, TNFRSF8, CCL18, CD163, TGFα or any combination thereof.

In a particular embodiment, the method may further comprise comparing the presence and/or amount of the combination of biomarkers obtained from step a) to the presence and/or amount of the same combination of biomarkers in a control sample; and classifying the individual as belonging to or not belonging to the control population, wherein the comparison determines a likelihood of upcoming delivery within N days after test day TD, and wherein the combination of biomarkers comprises, optionally consists of CCL2 and CEACAM1 and a combination of additional biomarkers consisting of CCL2, CEACAM1, IL1RL1, CD14, TNFRSF8, CCL18, CD163 and TGFα.

In one embodiment, the present invention relates to a method for prognosing upcoming delivery within N days after test day TD, the method comprising:
a) obtaining a sample of vaginal fluid from an individual,
b) determining the presence and/or amount of the combination of biomarkers using an antibody specific for the combination of biomarkers in the sample of step a), and
c) comparing the presence and/or amount of said combination of biomarkers obtained from step b) to the presence and/or amount of said combination of biomarkers in a control sample, to identify an increased risk for preterm delivery,
wherein the combination of biomarkers is one of the combinations of CCL2 and CEACAM1 and of any of the combinations of one or more of: IL1RL1, CD14, TNFRSF8, CCL18, CD163 and TGFα that are listed above.

The invention also provides kits or devices that are useful in predicting upcoming delivery.

The kits and devices of the present invention comprise at least the combination of biomarkers of the invention and/or one or more agent for the detection of or for the determination of the amount of the biomarkers of the combination of the invention. Specific biomarkers and agents for the detection of said biomarkers useful in the present invention are set forth herein.

Generally, the agents for the detection of the kit and the device will bind, with at least some specificity, to the biomarkers contained in the sample from which the combination of biomarkers is analyzed. Examples of classes of agents of the kit or device include, but are not to, proteins (including antibodies of the invention), and fragments thereof, peptides, polypeptides, proteoglycans, glycoproteins, lipoproteins, carbohydrates, lipids, nucleic acids, organic and inorganic chemicals, and natural and synthetic polymers. The agents for the detection of the biomarkers of the combination may be part of an array, or the agents may be packaged separately and/or individually. The agents may be immobilized on an inert support.

The kits and devices of the present invention also may contain reagents that can be used to detectably label biomarkers contained in the samples from which the combination of biomarkers is analyzed. For this purpose, the kit or device may comprise a set of antibodies or functional fragments thereof that specifically bind at least to CCL2 and CEACAM1 and optionally to one, two, three, four, five or six of the biomarkers selected among the group comprising IL1RL1, CD14, TNFRSF8, CCL18, CD163 and TGFα. The antibodies themselves may be detectably labelled. The kit or device also may comprise a specific biomarker binding component, such as an aptamer.

In one embodiment, the present invention further provides a device for carrying out the use of the invention or for use in the method of the invention, which comprises:
a) one or more antibody and/or oligonucleotide specific for the combination of CCL2 and CEACAM1 biomarkers for upcoming delivery, and
b) optionally, one or more antibody and/or oligonucleotide specific for the combination of one or more additional biomarkers for imminent birth, wherein the one or more additional biomarkers is selected from the group comprising IL1RL1, CD14, TNFRSF8, CCL18, CD163 and TGFα, and optionally
c) at least one internal standard.

As used herein, the at least one internal standard can be a negative control, such as stratifin (SFN).

If the biomarkers comprise a nucleic acid, the kit or device may provide one or more oligonucleotide probe that is capable of forming a duplex with the biomarkers of the combination or with a complementary strand of said biomarkers of the combination. The one or more oligonucleotide probe may be detectably labelled. Typically, the one or more oligonucleotide probe used in the methods of the invention is selected from one or more of the oligonucleotides described herein.

The kits and devices of the present invention may also include pharmaceutical excipients, diluents and/or adjuvants when the biomarker is to be used to raise an antibody. Examples of pharmaceutical adjuvants include, but are not limited to, preservatives, wetting agents, emulsifying agents, and dispersing agents. The kit may additionally include standards or controls. The kit may additionally include buffers, diluents or other reagents, such as stop buffer, sample preparation buffer, color development reagents, streptavidin conjugates, substrates or wash buffer.

The kit may comprise a device for obtaining or processing a vaginal fluid sample. The kit may comprise vaginal fluid extraction buffer, for example a buffer containing approximately 50mM HEPES, 150mM NaCl, 0.1 % SDS, 1 mM EDTA, 1 mM Pefabloc SC 4-(2-aminoethyl_benzene sulfonyl fluoride (AEBSF). The kit may comprise a sample collection device, such as a swab, cervicovaginal wick, diaphragm-like device, cervical aspirator, or cytobrush. The kit may comprise a container suitable for storing a vaginal fluid sample.

Prevention of the action of microorganisms can be ensured by the inclusion of various antibacterial and antifungal agents, for example, paraben, chlorobutanol, phenol sorbic acid, and the like. It may also be desirable to include isotonic agents such as sugars, sodium chloride, and the like.

In one embodiment, the kits or devices are described above are particularly useful in determining the risk of preterm delivery, optionally before 32 weeks of gestation.

In one embodiment, the kits or devices are described above are particularly useful:
- either for predicting preterm delivery if the test is performed at a test day TD at least N days before 37 weeks minus 1 day, N being positive and optionally ranging from 7 to 14 ;
- or for predicting spontaneous labor at term if the test is performed at a test day TD between N days before 37 weeks and N days before 41 weeks and 6 days, N being positive and optionally ranging from 7 to 14;
- or for predicting upcoming post term labor if the test is performed at a test day TD strictly after N days before 41 weeks and 6 days.

### EXAMPLES

### Example 1: evaluation of biomarkers combinations for delivery within N days after test day

### 1. Sample received and analyzed

### First cohort ("cohort 1"):

106 vaginal samples, obtained from women with symptomatic preterm labor, were analyzed for identifying specific biomarkers for preterm delivery. Among the 106 samples, 5 samples have been obtained from women who subsequently delivered within N = 7 days (respectively 10 samples within N = 14 days) after test day (case group) and 101 (respectively 96) who subsequently delivered after strictly more than N = 7 days (respectively strictly more than N = 14 days) after test day (control group).

### Second cohort ("cohort 2"):

120 vaginal samples, obtained from women with symptomatic preterm labor, were analyzed for identifying specific biomarkers for preterm delivery.

Among the 120 samples, 30 samples have been obtained from women who subsequently delivered within N = 7 days (respectively N = 36 samples within 14 days) after test day (case group) and 90 (respectively 84) who subsequently delivered after strictly more than N = 7 days (respectively strictly more than N = 14 days) after test day (control group).

### 2. Immunoassay analysis

### 2.1. COL1A1 Biomarker:

The MSD assay was set up according to the following protocol:
Coating: A High-Bind MSD large spot plate was spotted with 30µL per well of the capture (monoclonal) antibody at 2µg/ml in PBS. The plate was incubated at 4°C overnight without shaking. The plate was washed two times with 150µL per well of PBS + 0.05% Tween 20.
Blocking: A solution of 5% MSD blocker A in PBS was prepared for use as a blocking agent. 150µL of blocking buffer was added to all wells of the coated plate and the plate was incubated for 45 minutes at room temperature with vigorous shaking. The plate was then washed two times with 150µL of PBS + 0.05% Tween 20.
Sample: Vaginal samples was determined in PBS- 1% Blocker A and 25µL /well was loaded. The plate was incubated for 2 hours at room temperature with shaking before being washed three times with 150µL of PBS + 0.05% Tween 20.
Detection: Polyclonal antibody anti-COL1A1 at 0,1µg/ml in PBS + 1% Blocker A was added at 25µL /well. Plate was incubated for 2 hours at room temperature with shaking. Plate was then washed two times with 150µL of PBS + 0.05% Tween 20.
Streptavidin SulfoTAG: Streptavidin SulfoTAG was prepared at 0,5µg/ml in PBS + 1% Blocker A. 25µL of Streptavidin SulfoTAG reagent was added to all wells and the plate was incubated for 45 minutes at room temperature with shaking. Plate was then washed two times with 150µL of PBS+ 0.05% Tween 20.
Reading: 150µL of MSD Reading Buffer at 2x concentration was added to all wells and the plate was read immediately on the SectorImager 6000

### 2.2. CCN5 biomarker

The MSD assay was set up according to the following protocol:
Coating: A High-Bind MSD large spot plate was spotted with 30µL per well of the capture (monoclonal) antibody at 1µg/ml in PBS. The plate was incubated at 4°C overnight without shaking. The plate was washed two times with 150µL per well of PBS + 0.05% Tween 20.
Blocking: A solution of 5% MSD blocker A in PBS was prepared for use as a blocking agent. 150µL of blocking buffer was added to all wells of the coated plate and the plate was incubated for 45 minutes at room temperature with vigorous shaking. The plate was then washed two times with 150µL of PBS + 0.05% Tween 20.
Sample: Vaginal samples was determined in PBS- 1% Blocker A and 25µL /well was loaded. The plate was incubated for 2 hours at room temperature with shaking before being washed three times with 150µL of PBS + 0.05% Tween 20.
Detection: Polyclonal antibody anti-CCN5 at 0,4µg/ml in PBS + 1% Blocker A was added at 25µL /well. Plate was incubated for 2 hours at room temperature with shaking. Plate was then washed two times with 150µL of PBS + 0.05% Tween 20.
Streptavidin SulfoTAG: Streptavidin SulfoTAG was prepared at 0,5µg/ml in PBS + 1% Blocker A. 25µL of Streptavidin SulfoTAG reagent was added to all wells and the plate was incubated for 45 minutes at room temperature with shaking. Plate was then washed two times with 150µL of PBS+ 0.05% Tween 20.
Reading: 150µL of MSD Reading Buffer at 2x concentration was added to all wells and the plate was read immediately on the SectorImager 6000

### 2.3. CCL2, IL1RL1, CD14, TNFRSF8, CCL18, CD163, TGFα and CEACAM1 biomarkers

The Luminex assay was set up according to the following protocol:
50 µL of standard or sample was added per well. A plate layout is provided to record standards and samples assayed. The diluted Microparticle Cocktail was resuspended by inversion or vortexing.
50 µL of the microparticle cocktail was added to each well of the microplate. The microplate was then incubated for 2 hours at room temperature on a horizontal orbital microplate shaker (0.12" orbit) set at 800 ± 50 rpm.

A magnetic device designed to accommodate a microplate was used. Wash was realized by applying the magnet to the bottom of the microplate for 1 minute. The liquid was then removed. Each well was filled with Wash Buffer (100 µL) and the liquid was removed 1 minute later. The wash procedure was repeated three times. 50 µL of diluted Biotin-Antibody Cocktail was added to each well.

The plate was then incubated for 1 hour at room temperature on the shaker set at 800 ± 50 rpm. A magnetic device designed to accommodate a microplate was used. Wash was realized by applying the magnet to the bottom of the microplate for 1 minute. The liquid was then removed.

Each well was filled with Wash Buffer (100 µL) and the liquid was removed 1 minute later. The wash procedure was repeated three times. 50 µL of diluted Streptavidin-PE was added to each well. The plate was then incubated for 30 minutes at room temperature on the shaker set at 800 ± 50 rpm.

A magnetic device designed to accommodate a microplate was used. Wash was realized by applying the magnet to the bottom of the microplate for 1 minute. The liquid was then removed. Each well was filled with Wash Buffer (100 µL) and the liquid was removed 1 minute later. The wash procedure was repeated three times. Microparticles were resuspended by adding 100 µL of Wash Buffer to each well and incubate for 2 minutes on the shaker set at 800 ± 50 rpm.

The reading was realized within 90 minutes using a Luminex^{®} or Bio-Rad analyzer.

### 2.4. Identification of Stratifin (SFN) as internal standard

The MSD assay was set up according to the following protocol:
Coating: A High-Bind MSD large spot plate was spotted with 30µL per well of the capture (monoclonal) antibody at 2µg/ml in PBS. The plate was incubated at 4°C overnight without shaking. The plate was washed two times with 150µL per well of PBS + 0.05% Tween 20.
Blocking: A solution of 5% MSD blocker A in PBS was prepared for use as a blocking agent. 150µL of blocking buffer was added to all wells of the coated plate and the plate was incubated for 45 minutes at room temperature with vigorous shaking. The plate was then washed two times with 150µL of PBS + 0.05% Tween 20.
Sample: Vaginal samples was determined in PBS- 1% Blocker A and 25µL /well was loaded. The plate was incubated for 2 hours at room temperature with shaking before being washed three times with 150µL of PBS + 0.05% Tween 20.
Detection: Polyclonal antibody anti-SFN at 0,1µg/ml in PBS + 1% Blocker A was added at 25µL /well. Plate was incubated for 2 hours at room temperature with shaking. Plate was then washed two times with 150µL of PBS + 0.05% Tween 20.
Streptavidin SulfoTAG: Streptavidin SulfoTAG was prepared at 0,5µg/ml in PBS + 1% Blocker A. 25µL of Streptavidin SulfoTAG reagent was added to all wells and the plate was incubated for 45 minutes at room temperature with shaking. Plate was then washed two times with 150µL of PBS+ 0.05% Tween 20.
Reading: 150µL of MSD Reading Buffer at 2x concentration was added to all wells and the plate was read immediately on the SectorImager 6000

### Results:

Results are presented in Figure 2.

### Conclusion:

SFN protein was present in all the samples and SFN protein level was not statistically different between the group where the delivery birth was before 32 weeks gestation and the control group (see Figure 2). It thus results that SFN can be used as internal standard.

### 2.5. Dosage of fFN

Dosage of fetal fibronectin (fFN) and analyzed cervical length (CL) measurements performed at inclusion, when available.

Dosage of fetal fibronectin (fFN) was measured using the Human Fetal Fibronectin (fFN) ELISA of Cusabio^{®}. The assay procedure was implemented according to the instructions notice.

Reagents preparation: Biotin-antibody, HRP-avidin, and washing buffer were prepared according to the instructions notice of Human Fetal Fibronectin (fFN) ELISA of Cusabio^{®}.

Sample: Vaginal samples were collected and centrifuged for 15 minutes at 1000g at a temperature comprised between 2 and 8°C within 30 minutes. Then, the samples were assayed.

Briefly, reagent, samples and standards were prepared as instructed. 100µL of sample was added in each well and incubated for 2 hours at 37°C. The liquid was then removed from each well. 100µL of biotin-antibody was added in each well and incubated for 1 hour at 37°C. Each well was aspired and washed 3 times using a washing buffer. 100µL of HRP-avidin was added in each well and incubated for 1 hour at 37°C. Each well was aspired and washed 5 times using a washing buffer. 90µL of TMB Substrate was added in each well and incubated for 15 to 30 minutes at 37°C. 50µL of Stop solution was added in each well and the results were read at 450 nm within 5 minutes.

### TABLES

**Table 1 : Sensitivity and specificity of biomarkers combination with cohort 1 or cohort 2 for N = 7 days**

| **Prior art combination/cohort** | **Sensitivity** | **IC** | **Specificity** | **IC** | **TP** | **TN** | **FP** | **FN** |
|---|---|---|---|---|---|---|---|---|
| CCN5-COL1A1/ cohort 1 | 1.000 | [0.506-1.000] | 0.970 | [0.912-0.993] | 5 | 98 | 3 | 0 |
| CCN5-COL1A1/ cohort2 | 0.733 | [0.553-0.859] | 0.678 | [0.575-0.765] | 22 | 61 | 22 | 1 |
| IGFBP 1-IL6/cohort 2 | 0.800 | [0.622-0.907] | 0.900 | [0.818-0.948] | 24 | 81 | 6 | 6 |
| fFN/cohort 2 | 0.800 | [0.622-0.907] | 0.389 | [0.295-0.492] | 24 | 35 | 55 | 6 |

**Table 2a : Sensitivity and specificity of biomarkers combinations with cohort 2 for N = 7 days**

| **Combinations** | **Sensitivity** | **IC** | **Specificity** | **IC** | **TP** | **TN** | **FP** | **FN** |
|---|---|---|---|---|---|---|---|---|
| **CCL2-CEACAM1** | **0.933** | [0.779-0.992] | **0.756** | [0.654-0.840] | 28 | 68 | 22 | 2 |
| CCL2 | **0.933** | [0.774-0.991] | 0.733 | [0.633-0.814] | 28 | 66 | 24 | 2 |
| CEACAM1 | **0.933** | [0.774-0.991 ] | 0.278 | [0.196-0.379] | 28 | 25 | 65 | 2 |
| CCL2- IGFBP 1 | 0.8 | [0.622-0.907] | **0.922** | [0.845-0.964] | 24 | 83 | 7 | 6 |
| **CCL2-CEACAM1-**IGFPB1 | 0.8 | [0.622-0.907] | **0.937** | [0.858-0.971] | 24 | 84 | 6 | 6 |
| **CCL2-CEACAM1-**IGFPB1-IL6 | 0.8 | [0.622-0.907] | **0.933** | [0.858-0.971] | 24 | 84 | 6 | 6 |
| **CCL2-CFACAM1**-fFN | **0.933** | [0.774-0.991] | 0.744 | [0.645-0.823] | 28 | 67 | 23 | 2 |
| **CCL2-CEACAM1-**COL1A1-CCN5 | **0.933** | [0.774-0.991] | 0.589 | [0.486-0.685] | 28 | 53 | 37 | 2 |
| | | | | | | | | |
| **CCL2-CEACAM1 and** IL1RL1, CD14, TNFRSF8, CCL18, CD163 and TGFα | **0.967** | [0.816-1.000] | **0.789** | [0.692-0.861] | 29 | 71 | 19 | 1 |
| **CCL2-CEACAM1 and** IL1RL1, CD14, TNFRSF8, CCL18, CD163 and TGFα and COL1A1/CCN5 | **0.967** | **[0.828-0.999]** | 0.744 | **[0.642-0.831]** | 29 | 67 | 23 | 1 |

**Table 2b : Sensitivity and specificity of biomarkers combinations with cohort 1 for N = 7 days**

| **Biomarkers combinations** | **Sensibility** | **IC** | **Specificity** | **IC** | **TP** | **TN** | **FP** | **FN** |
|---|---|---|---|---|---|---|---|---|
| **CCL2-CEACAM1** | **1** | **[0.506-1.000]** | **1** | **[0.955-1.000]** | 5 | 101 | 0 | 0 |
| CCL2 | 1 | [0.506-1.000] | 0.99 | [0.940-1.000] | 5 | 100 | 1 | 0 |
| CEACAM1 | 0.6 | [0.231-0.88] | 0.822 | [0.734-0.885] | 3 | 83 | 18 | 2 |
| | | | | | | | | |
| **CCL2- CEACAM1** and IL1RL1, CD14, TNFRSF8, CCL18, CD163 and TGFα and COL1A1/CCN5 | 1 | [0.506-1.000] | 0.931 | [0.861-0.968] | 5 | 94 | 7 | 0 |
| **CCL2- CEACAM1** and IL1RL1, CD14, TNFRSF8, CCL18, CD163 and TGFα | 1 | [0.506-1.000] | **0.941** | [0.873-0.975] | 5 | 95 | 6 | 0 |

**Table 3 : Sensitivity and specificity of biomarkers combinations according to the invention with cohort 2 for N = 7 to 14 days**

| **Combinations** | **Sensitivity** | **IC** | **Specificity** | **IC** | **TP** | **TN** | **FP** | **FN** | **AUC** |
|---|---|---|---|---|---|---|---|---|---|
| **CCL2-CEACAM1** (<8davs) | **0.933** | [0.779-0.992] | **0.756** | [0.654-0.840] | 28 | 68 | 22 | 2 | 0.904 |
| **CCL2-CEACAM1** (<9davs) | 0.903 | [0.741-0.973] | 0.876 | [0.790-0.931] | 28 | 78 | 10 | 3 | 0.910 |
| **CCL2-CEACAM1** (<10days) | 0.906 | [0.748-0.974] | 0.886 | [0.801-0.938] | 29 | 78 | 10 | 3 | 0.909 |
| **CCL2-CEACAM1** (< 11 days) | 0.879 | [0.719-0.957] | 0.885 | [0.799-0.939] | 29 | 77 | 10 | 4 | 0.889 |
| **CCL2-CEACAM1** (<12days) | 0.882 | [0.726-0.958] | 0.895 | [0.810-0.945] | 30 | 77 | 9 | 4 | 0.894 |
| **CCL2-CEACAM1** (<13days) | 0.857 | [0.700-0.941] | 0.894 | [0.808-0.945] | 30 | 76 | 9 | 5 | 0.893 |
| **CCL2-CEACAM1** (<14days) | 0.833 | [0.676-0.924 | 0.893 | [0.806-0.944 | 30 | 75 | 9 | 6 | 0.878 |
| | | | | | | | | | |
| **CCL2- CEACAM1** and IL1RL1, CD14, TNFRSF8, CCL18, CD163 and TGFα (<8days) | **0.967** | [0.816-1.000] | **0.789** | [0.692-0.861] | 29 | 71 | 19 | 1 | 0.927 |
| **CCL2- CEACAM1** and IL1RL1, CD14, TNFRSF8, CCL18, CD163 and TGFα (<9days) | 0.903 | [0.741-0.973] | 0.831 | [0.739-0.896] | 28 | 74 | 15 | 3 | 0.897 |
| **CCL2- CEACAM1** and IL1RL1, CD14, TNFRSF8, CCL18, CD163 and TGFα (<10days) | 0.906 | [0.748-0.974] | 0.841 | [0.749-0.904] | 29 | 74 | 14 | 3 | 0.897 |
| **CCL2- CEACAM1** and IL1RL1, CD14, TNFRSF8, CCL18, CD163 and TGFα (<11days) | 0.909 | [0.755-0.975] | 0.839 | [0.746-0.902] | 30 | 73 | 14 | 3 | 0.896 |
| **CCL2- CEACAM1** and IL1RL1, CD14, TNFRSF8, CCL18, CD163 and TGFα (<12days) | 0.912 | [0.761-0.976] | 0.849 | [0.756-0.910] | 31 | 73 | 13 | 3 | 0.899 |
| **CCL2- CEACAM1** and IL1RL1, CD14, TNFRSF8, CCL18, CD163 and TGFα (<13days) | 0.914 | [0.767-0.977] | 0.859 | [0.767-0.918] | 32 | 73 | 12 | 3 | 0.900 |
| **CCL2- CEACAM1** and IL1RL1, CD14, TNFRSF8, CCL18, CD163 and TGFα ( <14days) | 0.889 | [0.739-0.961 ] | 0.857 | [0.764-0.917] | 32 | 72 | 12 | 4 | 0.900 |

## Claims

1. A combination of biomarkers comprising CCL2 and CEACAM1 for use as biomarkers of upcoming delivery of a pregnant individual within N days of a test day (TD), N being a strictly positive integer, optionally ranging from 7 to 14.

2. The combination of biomarkers according to claim 1, wherein the combination of biomarkers further comprises one or more additional biomarkers of upcoming delivery selected from the group comprising IL1RL1, CD14, TNFRSF8, CCL18, CD163 and TGFα.

3. The combination of biomarkers according to claim 1 to 2, wherein the one or more additional biomarkers comprise IL1RL1, CD14, TNFRSF8, CCL18, CD163 and TGFα.

4. The combination of biomarkers according to claim 1 to 3, wherein the combination of biomarkers comprises neither COL1A1 nor CCN5.

5. Use of a combination of biomarkers for prognosing and/or for diagnosing upcoming delivery of a pregnant individual within N days of a test day (TD), N being a strictly positive integer, optionally ranging from 7 to 14, wherein the combination of biomarkers comprises CCL2 and CEACAM1.

6. The use according to claim 5, wherein the combination of biomarkers further comprises one or more additional biomarkers of upcoming delivery selected from the group comprising IL1RL1, CD14, TNFRSF8, CCL18, CD163 and TGFα.

7. The use according to claim 5 to 6, wherein the combination of biomarkers comprises IL1RL1, CD14, TNFRSF8, CCL18, CD163 and TGFα.

8. A method for prognosing and/or for diagnosing upcoming delivery of a pregnant individual within N days of a test day (TD), N being a strictly positive integer, optionally ranging from 7 to 14, the method comprising:
a) determining at test day the presence and/or amount of a combination of biomarkers of upcoming delivery in a sample obtained from the individual, and
b) comparing the presence and/or amount of each biomarker of said combination of biomarkers obtained from step a) to the presence and/or amount of each biomarker of said combination of biomarkers in a control sample, to identify an increased risk for preterm delivery,
wherein said combination of biomarkers of upcoming delivery comprises CCL2 and CEACAM1.

9. The method according to claim 8, wherein the sample is a sample of vaginal fluid.

10. The method according to any one of claims 8 to 9, wherein the combination of biomarkers of upcoming delivery further comprises one or more additional biomarkers for upcoming delivery selected from the group comprising IL1RL1, CD14, TNFRSF8, CCL18, CD163 and TGFα.

11. The method according to any one of claims 8 to 10 or the use according to claims 5 to 7 or the combination of biomarkers according to claims 1 to 4, wherein the biomarkers are protein or nucleic acid encoding for said protein.

12. The method according to any one of claims 8 to 11, wherein the presence and/or amount of the CCL2, respectively CEACAM1, biomarker is determined using an antibody and/or an oligonucleotide specific for said CCL2, respectively CEACAM1, biomarker, and wherein the presence and/or amount of the one or more additional biomarkers for upcoming delivery is determined using an antibody and/or an oligonucleotide specific for said one or more additional biomarkers for upcoming delivery.

13. The method for prognosing and/or for diagnosing according to claims 8 to 12, wherein one more a) and one additional b) are performed at least at one time point (PTD) before test day (TD).

14. The use according to claims 5 to 7 or the method according to claim 8 to 13, wherein test day (TD) is comprised either at least N days before 37 weeks minus 1 day and upcoming birth is preterm birth ; test day (TD) is comprised between N days before 37 weeks and N days before 41 weeks and 6 days and upcoming birth is term birth; or test day (TD) is strictly after N days before 41 weeks and 6 days and upcoming birth is post term birth.

15. Use of a device for carrying out the use according to any one of claims 5 to 7, 11 and 14 or for carrying out the method according to any one of claims 8 to 13 and 14, which comprises:
a) one or more antibody and/or oligonucleotide specific for the combination of CCL2 and CEACAM1 biomarkers of upcoming delivery, and optionally
b) one or more antibody and/or oligonucleotide specific for any one combination of any of the one or more additional biomarkers of upcoming delivery selected from the group comprising IL1RL1, CD14, TNFRSF8, CCL18, CD163 and TGFα, and optionally
c) at least one internal standard.
